Europäisches Patentamt

(19) European Patent Office

Office européen des brevets

(11) Publication number: **0 021 479**
**B1**

(12) **EUROPEAN PATENT SPECIFICATION**

(45) Date of publication of patent specification: **08.12.82**

(21) Application number: **80200503.3**

(22) Date of filing: **28.05.80**

(51) Int. Cl.³: **C 07 C 7/10, C 07 C 7/148**
**//C07C9/08**

(54) Process and plant for the removal of acidic components.

(30) Priority: **11.06.79 GB 7920182**

(43) Date of publication of application:
**07.01.81 Bulletin 81/1**

(45) Publication of the grant of the patent:
**08.12.82 Bulletin 82/49**

(84) Designated Contracting States:
**DE FR GB NL**

(56) References cited:
**DE - A - 2 754 797**
**GB - A - 1 501 294**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Van Grinsven, Petrus Franciscus Antonius**
**Badhuisweg 3**
**NL-1031 CM Amsterdam (NL)**

(74) Representative: **Puister, Antonius Tonnis, Mr. et al,**
**P.O. Box 302**
**NL-2501 CH The Hague (NL)**

Courier Press, Leamington Spa, England.

## Process and plant for the removal of acidic components

The invention relates to a continuous process for the removal of acidic components from a liquid feed stream consisting for the greater part of hydrocarbons with 3—6 carbon atoms per molecule, which process comprises contacting the said liquid feed stream in an extractor with a water-containing solvent which contains a physical absorbent and/or a chemical absorbent, removing loaded solvent from the extractor and regenerating it before bringing it into contact again with the said feed stream.

For the purpose of this specification, carbonyl sulphide (COS) and mercaptans (RSH) will be considered to be acidic components in addition to $H_2S$ and $CO_2$.

A process of the type mentioned in the beginning is described in German published patent application 2,754,797. This known process involves removal of carbonyl sulphide from a liquid propane stream by contacting the stream with diglycolamine containing 10—100% by weight of diglycolamine (at most 90% by weight of water). Aqueous solutions containing at least 15% by weight of diglycolamine (at most 85% by weight of water) are most effective for the removal of carbonyl sulphide.

The Applicants have realized that use of the latter solutions may give rise to problems. Application of solvents containing more than 40% by weight, for example 65 to 75% by weight of water has indeed the advantage that the hydrocarbons with 3—6 carbon atoms are not absorbed in an appreciable amount, but has the disadvantage that mercaptans, and, in the presence of many solvents, carbonyl sulphide, are not absorbed in an appreciable amount; accordingly, a separate step for the removal of mercaptans and carbonyl sulphide was necessary. Application of solvents containing at most 40% by weight of water has the disadvantage that the hydrocarbons with 3—6 carbon atoms are also absorbed by the solvent; this disadvantage involves unacceptable losses of hydrocarbons.

It is an object of the present invention to remove the acidic components and to prevent unacceptable losses of hydrocarbons due to absorption thereof in the solvent.

Accordingly, the invention provides a continuous process for the removal of acidic components from a liquid feed stream consisting for the greater part of hydrocarbons with 3—6 carbon atoms per molecule, which process comprises contacting the said liquid feed stream in an extractor with a water-containing solvent which contains a physical absorbent and/or a chemical absorbent, removing loaded solvent from the extractor and regenerating it before bringing it into contact again with said feed stream, characterized in that the said solvent contains at most 30% by weight of water, entrained or absorbed hydrocarbons are separated from the loaded solvent at reduced pressure in a flash-vessel before regeneration of the loaded solvent and the hydrocarbons set free in the flash-vessel are returned to the extractor after being reliquefied.

The solvent with which the feed stream is to be contacted preferably contains at most 20% w of water.

A physical absorbent in the context of this specification is a compound in which acid gases are soluble without reacting therewith. Examples of physical absorbents are aliphatic acid amides (e.g. dimethylformamide), N-alkylated pyrrolidones (e.g. N-methylpyrrolidone), N-alkylated piperidones, dialkylethers of polyalkylene glycols (e.g. dimethyl ether of polyethylene glycol), cyclotetramethylenesulphones, in particular tetrahydrothiophene 1,1-dioxide (also named "sulfolane"), are preferred.

A chemical absorbent in the context of this specification is a compound which is able to react chemically with acid gases. Examples of chemical absorbents are primary, secondary and tertiary amines; mixtures of these types of amines may also be used. Very suitable are amines which contain at least one hydroxyalkyl group; examples are ethanolamine, diethanolamine, triethanolamine, methyldiethanolamine, diethylethanolamine, and in particular diisopropanolamine.

The feed stream very suitably consists of hydrocarbon mixtures which have been obtained as a condensate from natural gas by cooling; hydrocarbons with more than 2 carbon atoms per molecule have to be separated from natural gas in order to make the latter suitable for pipe line transport and/or condensation.

Other very suitable feed streams for the present process consist entirely or for the greater part of propane or butane or mixtures thereof, e.g., those known in the art as LPG.

The loaded solvent which leaves the extractor is flashed in a flash-vessel at reduced pressure. It is preferred to release the pressure before the loaded solvent enters the flash-vessel.

In order to obtain a very effective release of absorbed hydrocarbons from the loaded solvent, and in particular where all or part of the hydrocarbons in question have a higher boiling point than the extraction temperature at the pressure obtained after the pressure reduction step, the loaded solvent stream leaving the extractor may be heated e.g. by bringing it into heat exchange contact with the regenerated solvent prior to passing the loaded solvent to the flash-vessel.

The hydrocarbons released in the flash-vessel are advantageously routed via a small absorber in which they are contacted with a by-pass stream of the lean solvent prior to relique- faction which is preferably accomplished by recompression and cooling.

This additional absorption step can often be justified because when the hydrocarbons are flashed off from the loaded solvent, particularly when the temperature of the loaded solvent has been increased prior to flashing, absorbed acidic components are also released. The hydrocarbons recovered from the flash-vessel are then likely to contain an abnormally high proportion of acidic components which can be more economically reduced to the same order of magnitude as that of the feed stream by a pre-treatment in the small absorber than in the extractor, as would be the case if they were mixed directly back into the main stream.

It has further been found of advantage to wash the treated hydrocarbons which leave the extractor. This washing may be carried out in one or more stages, and the water which is used may be added to the stream of loaded solvent, conveniently upstream of the regenerator, preferably upstream of the flash-vessel.

In view of the low water contents of the solvent, regeneration can be a delicate operation requiring elaborate controls in order to maintain the regeneration temperature within the required range due to the inherently low vapour pressure of the solvent. Also for that reason the addition of the wash water of the treated hydrocarbons to the loaded absorbent upstream of the regenerator (as discussed above) is of advantage.

Alternatively the said wash water can be passed directly to the regenerator and introduced at or near the bottom of the regenerator or to a reboiler associated with it.

Regeneration of the loaded solvent in the regenerator is carried out by heating. This heating may be accomplished with live steam, but is preferably carried out with the aid of a reboiler.

The acid gases, and absorbed hydrocarbons (if still present) are released at the top of the regenerator, together with some water, which leaves as a vapour and is subsequently recondensed. It will often be desirable to steam strip this water to remove traces of $H_2S$ and mercaptans which may be dissolved in it. It is then preferably returned to the water wash step of the treated hydrocarbons discussed above.

It has been found that by using a high solvent-to-hydrocarbons ratio, conveniently from 1:1 to 10:1, a very effective removal of mercaptans can be obtained. However, in the absence of the invention, there would be such a high co-absorption of the hydrocarbons that any savings in the plant, and in solvent costs would be more than offset by the substantial loss of hydrocarbons in the solvent. For example, a solvent containing 5% water may absorb some 5% w propane, and if a solvent: hydrocarbons ratio of, say, 5:1 is maintained, some 25% w propane could be lost. However, by applying the method of the invention, the propane can be very largely recovered. Furthermore, by recovering it before it enters the regenerator there is only a small quantity of hydrocarbons which find their way to the sulphur recovery unit where the acidic components from the regeneration are treated. This results in a better quality of sulphur, particularly regarding its colour.

For effective mercaptan removal, the solvent : hydrocarbons ratio should be in excess of 1 : 1 and preferably in excess of about 2 : 1. Where a solvent with a very low water content is employed, this ratio cannot be increased very much above this figure, but for solvents containing more water it may be advantageous to increase the ratio up to 5 : 1. Above this level the cost of stripping per ton of hydrocarbons treated is likely to become excessive, and may exceed any savings made due to the omission of a separate mercaptan removal plant.

The invention also relates to a plant for carrying out the process in accordance with the invention. Such plant comprises an extractor in which hydrocarbons to be treated can be contacted with a solvent and operatively arranged so that loaded solvent from the extractor can be passed to a regenerator where it can be regenerated for reuse. The plant is characterized in that in the line leading from the extractor to the regenerator there is a pressure reduction device and a flash-vessel arranged to operate at a lower pressure than the extractor. The gas outlet of the flash-vessel is connected to the extractor via compressor.

Additionally, the plant may be provided with one or more water wash vessels in the line for treated hydrocarbons leading from the extractor. The water used in the water wash vessels is coveniently that which is condensed at the outlet of the regenerator. Means may be provided for stripping acidic components from the condensate upstream of the water wash vessel(s).

The invention will now be described by way of example with reference to the accompanying drawings.

Figure 1 is a schematic block diagram of a plant in accordance with the invention;

Figure 2 is a schematic block diagram of an alternative embodiment of a plant in accordance with the invention; and

Figure 3 is a schematic block diagram of a further alternative embodiment of a plant in accordance with the invention.

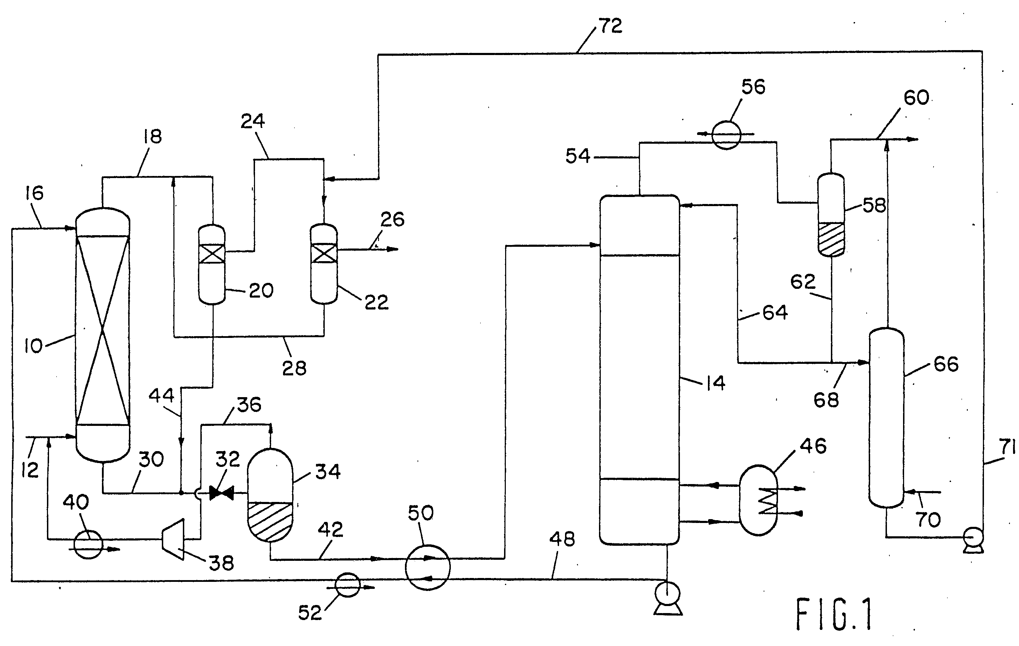

More specifically, Figure 1 shows a plant for treating LPG (which consists predominantly of propane and butane) for removing acidic components which it contains. The plant basically comprises an extractor 10 which the LPG to be treated enters in the liquid phase via line 12, and a regenerator 14 where solvent used to treat the LPG in the extractor 10 is regenerated.

As shown the extractor 10 is a packed column which the LPG enters at its lower end and progresses upwardly through the packing, e.g. raschig rings, in counter flow with solvent which enters the column near the top by line 16.

Treated LPG leaves the extractor at its upper end by line 18 by which it passes to two water wash-vessels 20, 22 arranged in series. Wash water is added directly to the LPG in line 24 between the two vessels.

The washed LPG leaves the second water wash vessel 22 by line 26, whilst the separated water is removed from the bottom of the vessel by line 28 by which it is injected into the line 18 upstream of the first water wash vessel 20.

As stated the solvent flows downwardly in the extractor 10 and leaves it by the bottom by line 30. After being reduced in pressure in a throttle 32 from the high pressure obtaining in the extractor, say, 30 to 45 bar, to, say, 1.5 to 2 bar, which is the pressure in the regenerator, it enters a flash-vessel 34. LPG leaving the upper end of the flash-vessel by line 36 is recompressed to slightly above the extractor pressure by a compressor 38, cooled in a cooler/condenser 40 and is added back to the LPG inlet line 12 at the bottom of the extractor.

The fat solvent leaves the flash-vessel 34 at its lower end by line 42 leading to the regenerator 14 which it enters near the top. Water from the water wash vessel 20 is combined with the fat solvent in line 30.

The regenerator 14 typically comprises a vertical column having a plurality of trays (not shown) which hold up the downward flow of the solvent while steam rises through the trays from the bottom of the column where the solvent is reboiled by a reboiler 46.

Regenerated solvent leaves the regenerator by the bottom and is recirculated by line 48 to the extractor having been cooled to the extractor operating temperature, generally between 30 and 60°C, by heat exchange with the cooler fat solvent in a heat exchanger 50, and further by a cooler 52.

The acidic components released from the solvent in the regenerator, together with some steam, leave the top of the regenerator by line 54. After cooling in a cooler 56 the acidic components, mainly $H_2S$, COS and mercaptans are separated from the condensate in a separator 58 and leave the plant for further treatment by line 60. The condensate is removed from the bottom of the separator by line 62 and is partly recirculated to the top of the regenerator by line 64 as wash water for the gases and vapour leaving the regenerator, and partly to a sour water stripper 66 by line 68. The acidic components are removed from the condensate by direct steam injection into the stripper 66 via line 70. The resulting purified water is then recirculated to the the LPG water wash vessels through line 72.

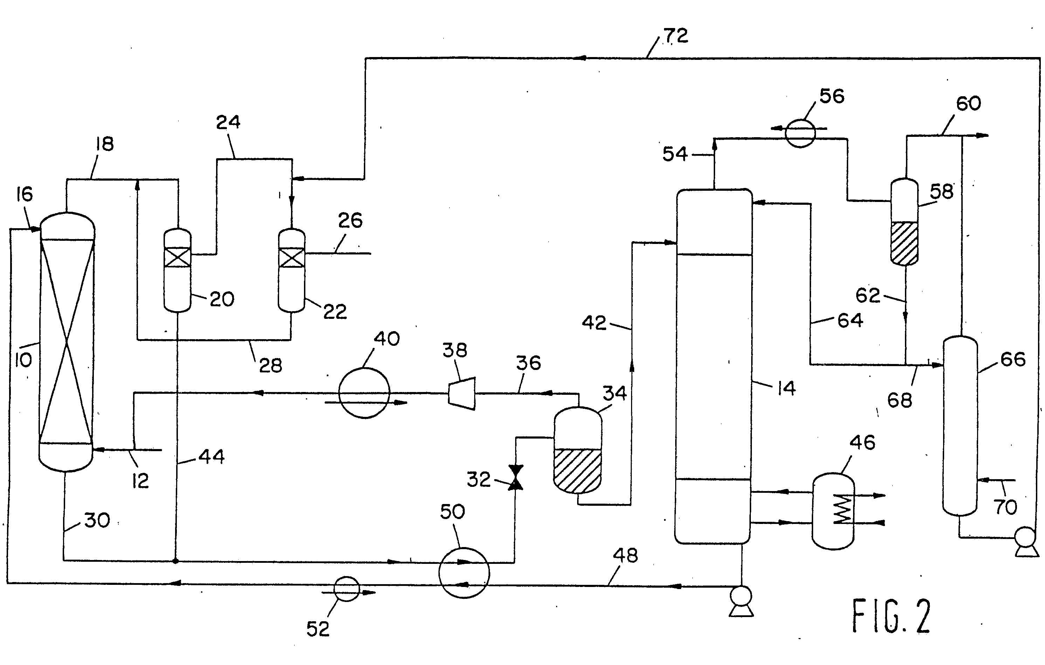

The plant shown in figure 2 is substantially identical to that shown in figure 1 with the exception that the throttle 32 and the flash vessel 34 are placed down-stream of the heat exchanger 50 so that the fat solvent leaving the extractor in the line 30 is heated by the warmer regenerated solvent in the line 48 from the regenerator 14 prior to its entering the flash-vessel. In his case the wash water from the water wash-vessel 20 is combined with the fat solvent in line 30 upstream of the flash-vessel. This embodiment may be more suitable for separating of the absorbed hydrocarbons in the fat solvent when the former have a higher boiling point.

The circuit shown in figure 1 is particularly suitable for propane, whereas the one in figure 2 may also be suited to the treatment of light gasolines.

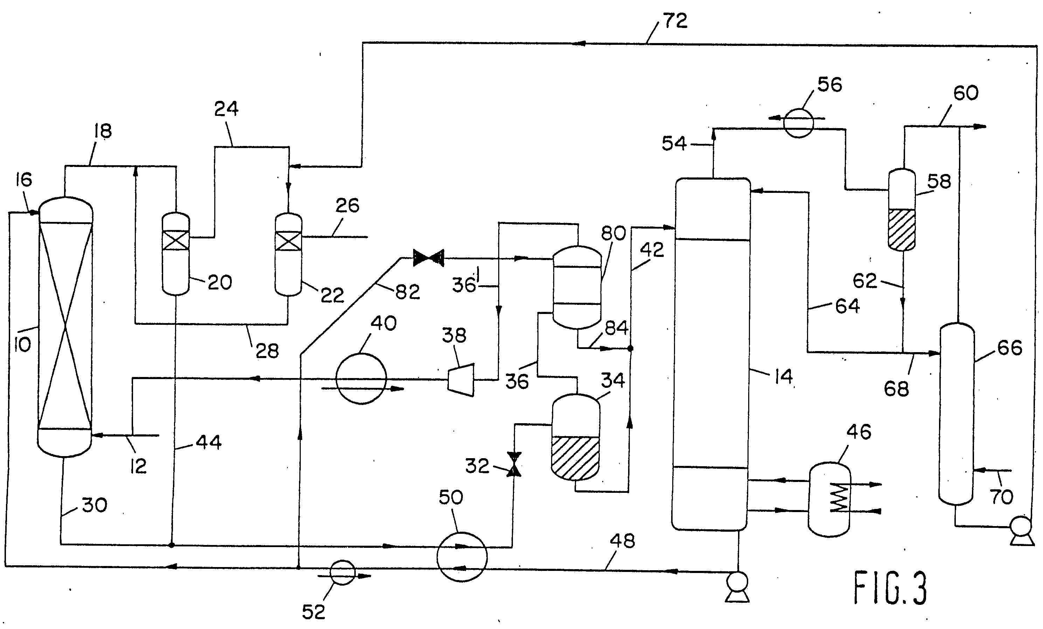

The circuit shown in figure 3 is basically similar to the one shown in figure 2 but that an acidic component removal facility is interposed in the LPG outlet line 36 from flash-vessel 34 so that the LPG which is recirculated to the extractor does not contain an abnormally high proportion of acidic components, which might otherwise be the case. This acidic component removal facility comprises a small absorber column 80, conveniently of the tray type, which is fed with a by-pass stream of lean, cooled solvent by line 82 from the main lean solvent line 48. The solvent enters the column 80 near the top and flows downwardly in counter-current to the rising LPG which, treated, leaves by the line 36[1] leading to the LPG inlet 12 to the extractor 10. The loaded solvent is passed directly to the regenerator 14 via lines 84 and 42. Due to the high acidic component content of the LPG leaving the flash-vessel 34 the solvent flow in this recontractor is small and therefore the column 80 need not be large.

Whilst the extractors shown in Figures 1, 2 and 3 are of the column type, each or any of them may equally comprise one or more mixer vessels each provided with a stirrer and having one or more settlers for separating the hydrocarbons from the solvent. Such an arrangement has an advantage over the column shown where a longer residence time is required for the reaction, such as for example for mercaptans removal, and also may be advantageous where high solvent : hydrocarbons ratios are to be employed.

Example

Referring to Figure 2, propane containing acidic components comprising 1.5% w $H_2S$, 600 ppm COS and 300 ppm methyl mercaptan ($CH_3SH$). The treated propane leaving the plant contains 0.5 ppm $H_2S$, 3 ppm COS and 5 ppm $CH_3SH$.

The solvent composition in the extractor is as follows:
45 parts wt di-isopropylamine (DIPA)
40 parts wt sulfolane

The solvent to propane ratio is 4 : 1 by weight in the extractor. The pressure is approximately 20 bar and the temperature between 40 and 45°C.

The propane leaving the extractor contains 0.08% w DIPA and 0.72% w sulfolane, which is recuded to a total of 10 ppm after the two washing steps 20 and 22. Wash water is added to the propane stream via line 24 in the water : propane ratio 1 : 4. When this water with the solvent recovered in the wash steps is added to the main solvent stream the effect is to increase the proportion of water in the solvent from 15 to 20 parts wt, the other proportions remaining unchanged.

The loaded solvent leaving the extractor contains 3% w propane and, after heat exchange with the regenerated solvent and throttling, is flashed in the flash-vessel 34 at a temperature of 113°C at approximately 2 bar abs. The solvent leaving the flash-vessel contains a maximum of about 0.1% w propane.

When an absorber 80 (Fig. 3) is placed in the propane outlet from the flash-vessel 34, 0.2 part wt solvent per part wt propane is sufficient to reduce its acidic component content to a suitable level for it to be returned to the inlet 12 of the extractor 10.

## Claims

1. A continuous process for the removal of acidic components from a liquid feed stream consisting for the greater part of hydrocarbons with 3—6 carbon atoms per molecule, which process comprises contacting the said liquid feed stream in an extractor with a water-containing solvent which contains a physical absorbent and/or a chemical absorbent, removing loaded solvent from the extractor and regenerating it before bringing it into contact again with the said feed stream, characterized in that the said solvent contains at most 30% by weight of water, entrained or absorbed hydrocarbons are separated from the loaded solvent at reduced pressure in a flash-vessel before regeneration of the loaded solvent and the hydrocarbons set free in the flash-vessel are returned to the extractor after being reliquefied.

2. A process as claimed in claim 1 in which the solvent contains at most 20% w of water.

3. A process as claimed in claim 1 or 2 in which the temperature of the loaded solvent is increased prior to its entering the flash vessel.

4. A process as claimed in any one of the preceding claims in which the hydrocarbons are reliquefied by compression and cooling.

5. A process as claimed in any one of the preceding claims in which the hydrocarbons leaving the flash-vessel are contacted with a by-pass stream of the said solvent prior to being returned to the extractor.

6. A process as claimed in any one of the preceding claims in which the stream of treated hydrocarbons is washed with water after leaving the extractor.

7. A process as claimed in claim 6 in which the wash water is added to the stream of loaded solvent upstream of the flash-vessel.

8. A process as claimed in claim 6 or 7 in which water vapour leaving the regenerator is condensed and the resulting condensate is circulated to the water wash.

9. A process as claimed in any one of the preceding claims in which the solvent : hydrocarbons ratio in the extractor is between 1 : 1 and 5 : 1.

10. Plant for carrying out the process claimed in any one of the preceding claims comprising an extractor arranged so that a stream of hydrocarbons entering it is contacted by a solvent which solvent on leaving the extractor is regenerated in a regenerator which is operatively connected with the extractor for recirculation of the solvent in a solvent circuit, characterized in that the line leading from the extractor in the solvent circuit is provided with a pressure reducing device and a flash-vessel arranged to operate at a lower pressure than the extractor, and whose gas outlet is connected to the extractor via a compressor.

## Revendications

1. Un procédé continu pour l'élimination de composants acides d'un courant de charge liquide constitué en majeure partie d'hydrocarbures ayant de 3 à 6 atomes de carbone par molécule, selon lequel on met en contact le courant de charge liquide dans un extracteur avec un solvant contenant de l'eau qui contient un absorbant physique et/ou un absorbant chimique, on évacue le solvant chargé de l'extracteur et on le régénère avant de le mettre en contact de nouveau avec le courant de charge, caractérisé en ce que ledit solvant contient au maximum 30% en poids d'eau, que les hydrocarbures entraîns ou absorbés sont séparés du solvant chargé sous pression réduite dans un récipient de détente avant régénération du solvant chargé et que les hydrocarbures libérés dans le récipient de détente sont ramenés à l'extracteur après avoir été reliquéfiés.

2. Un procédé selon la revendication 1, dans lequel le solvant contient au maximum 20% en poids d'eau.

3. Un procédé selon la revendication 1 ou 2, dans lequel la température du solvant chargé est élevée avant son introduction dans le récipient de détente.

4. Un procédé selon l'une quelconque des revendications précédentes, dans lequel les hydrocarbures sont reliquéfiés par compression et refroidissement.

5. Un procédé selon l'une quelconque des revendications précédentes, dans lequel les hydrocarbures quittant le récipient de détente sont mis en contact avec un courant en dérivation du solvant avant d'être ramenés à l'extracteur.

6. Un procédé selon l'une quelconque des revendications précédentes, dans lequel le courant d'hydrocarbures traités est lavé à l'eau après avoir quitté l'extracteur.

7. Un procédé selon la revendication 6, dans lequel l'eau de lavage est ajoutée au courant de solvant chargé en amont du récipient de détente.

8. Un procédé selon la revendication 6 ou 7, dans lequel la vapeur d'eau quittant le régénérateur est condensée et le condensat résultant est recyclé à l'eau de lavage.

9. Un procédé selon l'une quelconque des revendications précédentes, dans lequel le rapport solvant : hydrocarbures dans l'extracteur est compris entre 1 : 1 et 5 : 1.

10. Un installation utilisable pour mise en oeuvre du procédé selon l'une quelconque des revendications précédentes, comprenant un extracteur agencé de manière qu'un courant d'hydrocarbures qui y entre soit mis en contact avec un solvant, lequel solvant à sa sortie de l'extracteur est régénéré dans un régénérateur qui est relié de manière appropriée à l'extracteur pour recyclage du solvant dans un circuit de solvant, caractérisée en ce que la canalisation venant de l'extracteur dans le circuit de solvant est pourvue d'un dispositif réducteur de pression et d'un récipient de détente prévu pour fonctionner à une pression plus basse que l'extracteur, et dont la sortie pour gaz est reliée à l'extracteur par l'intermédiaire d'un compresseur.

## Patentansprüche

1. Ein kontinuierliches Verfahren zum Entfernen saurer Komponenten aus einem flüssigen Beschickungsstrom, welcher zum grösseren Teil aus Kohlenwasserstoffen mit 3 bis 6 Kohlenstoffen je Molekül besteht, welches Verfahren ein In-Berührung-Bringen des genannten flüssigen Beschickungsstroms in einem Extraktor mit einem wasserhaltigen, ein physikalisches Absorptionsmittel und/oder ein chemisches Absorptionsmittel enthaltenden Lösungsmittel, ein Entfernen von beladenem Lösungsmittel aus dem Extraktor und ein Regenerieren desselben umfasst, bevor dieses wieder mit dem genannten Beschickungsstrom in Berührung gebracht wird, dadurch gekennzeichnet, dass das genannte Lösungsmittel höchstens 30 Gewichtsprozent Wasser enthält, mitgeführte oder absorbierte Kohlenwasserstoffe vor dem Regenerieren des beladenen Lösungsmittels unter vermindertem Druck in einem Schnellverdampfer aus dem beladenen Lösungsmittel entfernt werden und die in dem Schnellverdampfer freigesetzten Kohlenwasserstoffe nach der Wiederverflüssigung in den Extraktor rückgeführt werden.

2. Ein Verfahren wie in Anspruch 1 beansprucht, in dem das Lösungsmittel höchstens 20 Gewichtsprozent Wasser enthält.

3. Ein Verfahren wie in Anspruch 1 oder 2 beansprucht, in dem die Temperatur des beladenen Lösungsmittels vor dessen Eintreten in den Schnellverdampfer erhöht wird.

4. Ein Verfahren wie in irgendeinem der vorangehenden Ansprüche beansprucht, in dem die Kohlenwasserstoffe durch Komprimieren und Abkühlen wiederverflüssigt werden.

5. Ein Verfahren wie in irgendeinem der vorangehenden Ansprüche beansprucht, in dem die aus dem Schnellverdampfer austretenden Kohlenwasserstoffe vor der Rückführung in den Extraktor mit einem Umgehungsstrom des genannten Lösungsmittels in Berührung gebracht werden.

6. Ein Verfahren wie in irgendeinem der vorangehenden Ansprüche beansprucht, in dem der Strom behandelter Kohlenwasserstoffe nach dem Austreten aus dem Extraktor mit Wasser gewaschen wird.

7. Ein Verfahren wie in Anspruch 6 beansprucht, in dem das Waschwasser dem Strom an beladenem Lösungsmittel vor dem Schnellverdampfer zugesetzt wird.

8. Ein Verfahren wie in Anspruch 6 oder 7 beansprucht, in dem den Regenerator verlassender Wasserdampf kondensiert wird und das entstehende Kondensat im Kreislauf zur Wasserwäsche rückgeführt wird.

9. Ein Verfahren wie in irgendeinem der vorangehenden Ansprüche beansprucht, in dem das Verhältnis von Lösungsmittel : Kohlenwasserstoffen in dem Extraktor zwischen 1 : 1 und 5 : 1 beträgt.

10. Anlage zur Durchführung des in irgendeinem der vorangehenden Ansprüche beanspruchten Verfahrens, umfassend einen Extraktor, welcher so angeordnet ist, dass ein in diesen eintretender Strom von Kohlenwasserstoffen durch ein Lösungsmittel berührt wird, welches Lösungsmittel bei seinen Austreten aus dem Extraktor in einem Regenerator regeniert wird, welcher mit dem Extraktor zur Rückführung des Lösungsmittels in einem Lösungsmittelkreislauf betriebsmässig verbunden ist, dadurch gekennzeichnet, dass die von dem Extraktor in den Lösungsmittelkreislauf führende Leitung mit einer Druckminderungsvorrichtung und mit einem Schnellverdampfer versehen ist, der so ausgelegt ist, dass er bei geringerem Druck als der Extraktor arbeitet und dessen Gasauslass mit dem Extraktor über einem Kompressor verbunden ist.

FIG.1

FIG. 2

0021 479

FIG.3

0021479